# EUROPEAN PATENT SPECIFICATION

(11) **EP 4 337 300 B1**
(45) Date of publication and mention of the grant of the patent: **08.07.2026**
(21) Application number: 22808386.1
(22) Date of filing: 13.05.2022
(51) Int. Cl.: A61M 39/10, A61M 25/00, A61M 5/158

(54) **CATHETER ASSEMBLY HAVING A SIDE PORT PATHWAY**
KATHETERANORDNUNG MIT SEITENPORTWEG
ENSEMBLE CATHÉTER COMPORTANT UN PASSAGE D'ORIFICE LATÉRAL

(30) Priority: 14.05.2021 US 202163188834 P
(43) Date of publication of application: 20.03.2024
(60) Divisional application: 26179763.3
(73) Proprietor: Becton, Dickinson and Company, Franklin Lakes, NJ 07417 (US)
(72) Inventor: LACKEY, John, West Valley City, Utah 84119 (US); BURKHOLZ, Jonathan, Karl, Salt Lake City, Utah 84108 (US); MA, Yiping, Layton, Utah 84040 (US); LACKEY, BreAnna, West Valley City, Utah 84119 (US); SONDEREGGER, Ralph, L., Farmington, Utah 84025 (US); MITCHELL, Nathan, Murray, Utah 84123 (US); SCHERICH, Megan, Salt Lake City, Utah 84115 (US); BLANCHARD, Curtis, H., Riverton, Utah 84096 (US); BOUD, Adam, J., Bluffdale, Utah 84065 (US); ELEY, Taylor, Alpharetta, Georgia 30005 (US); LAUER, Shaun, Portland, Oregon 97203 (US)
(74) Representative: dompatent
(86) International application number: PCT/US2022/029141
(87) International publication number: WO 2022/241189

(56) References cited:
- AU-A1- 2016 346 888
- CA-A1- 3 137 501
- US-A- 5 053 004
- US-A1- 2012 191 029
- US-A1- 2017 281 922
- US-A1- 2018 154 112
- US-A1- 2019 022 357
- US-A1- 2019 022 357
- US-A1- 2019 091 462
- US-A1- 2020 197 682
- US-A1- 2020 376 234
- US-A1- 2021 128 037

## Description

### CROSS-REFERENCE TO RELATED APPLICATION

The present application claims priority to United States Provisional Application Serial No. 63/188,834, entitled "Catheter Assembly Having a Side Port Pathway and Related Methods", filed May 14, 2021.

### BACKGROUND OF THE INVENTION

A catheter is commonly used to infuse fluids into vasculature of a patient. For example, the catheter may be used for infusing normal saline solution, various medicaments, or total parenteral nutrition. The catheter may also be used for withdrawing blood from the patient.

The catheter may include an over-the-needle peripheral intravenous ("IV") catheter. In this case, the catheter may be mounted over an introducer needle having a sharp distal tip. The catheter and the introducer needle may be assembled so that the distal tip of the introducer needle extends beyond the distal tip of the catheter with the bevel of the needle facing up away from skin of the patient. The catheter and introducer needle are generally inserted at a shallow angle through the skin into vasculature of the patient.

In order to verify proper placement of the introducer needle and/or the catheter in the blood vessel, a clinician generally confirms that there is "flashback" of blood in a flashback chamber of a catheter assembly that includes the catheter. After placement of the needle has been confirmed, the clinician may remove the needle, leaving the catheter in place for future blood withdrawal or fluid infusion.

Many catheter assemblies have a septum proximal to an extension tube inlet, creating a region distal to the septum that is difficult to flush free of fluids (such as blood or infusates). Stagnant fluid within the region distal to the septum may lead to accumulation of bacteria within the catheter assembly, which may result in infection or removal of the catheter from the patient.

AU 2016346888 A1 discloses: a compliant catheter adapter including a catheter adapter body formed of a compliant material. The catheter adapter body may have a generally elongate shape and an inner chamber. The catheter adapter body includes a compression resistant septum disposed toward the proximal end of the catheter adapter body. The compression resistant septum has a lumen configured to receive an elongate object. The compression resistant septum may also be coupled to a compression cap that imparts a compression force on the compression resistant septum such that the lumen narrows and seals when the elongate object is removed from the lumen.

CA 3137501 A1 discloses a support device to support a catheter assembly including a platform, which includes an upper surface and a bottom surface. The support device includes an extension element coupled to the upper surface of the platform. The extension element includes a distal end, which may include a first connector configured to couple to the catheter assembly, and a proximal end, which may include a second connector. The extension element may include a valve that may be movable between a first position and a second position. In response to the valve being moved to the first position, a fluid pathway extending through the cannula and the extension element may be open and straight. In response to the valve being moved to the second position, the fluid pathway may be closed. The support device may include a cannula, which may be swaged within the extension element.

US 5,053,004 A discloses a double-lumen catheter made of two generally coaxial tubes. The tubes are joined, at their distal ends, by a sleeve inserted between the tubes, the sleeve being bonded to both tubes. The inner and outer tubes are connected, respectively, at their proximal ends, to first and second hubs. The hubs are bonded together. The distal ends of the tubes may be formed into a tapered tip, preferably having a rounded edge. The proximal ends of the tubes can be connected to external fluid devices.

US 2019/022357 A1 discloses a vascular access device. The vascular access device includes a catheter and catheter adapter having a catheter hub and side port. The contact angle of a probe is greater than 90 degrees. The entrance angle of a probe entering the catheter hub from the side port may be less than 45 degrees. The vascular access device may include a component configured to direct the path of a probe towards the catheter opening. The component may be a protrusion extending into the lumen of the side port, internal fluid passageway, and/or extension tube, or a septum within the catheter hub. The vascular access device includes an access adapter in fluid communication with the side port and permitting insertion of a probe into the catheter through the side port with or without a separate luer adapter.

The subject matter claimed herein is not limited to embodiments that solve any disadvantages or that operate only in environments such as those described above. Rather, this background is only provided to illustrate one example technology area where some implementations described herein may be practiced.

### SUMMARY OF THE INVENTION

The invention is defined in the claims. The present disclosure relates generally to vascular access devices, and systems. More particularly, the present disclosure relates to a catheter assembly having a side port pathway. According to the present invention, the catheter assembly includes a catheter adapter, which includes a distal end, a proximal end, and an inner surface extending through the distal end and the proximal end. The inner surface of the catheter adapter forms a lumen. The catheter adapter includes a side port forming a side port pathway through a sidewall of the catheter adapter and in fluid communication with the lumen.

In some embodiments, the catheter assembly may facilitate flushing of the catheter assembly to avoid stagnant fluid within the catheter assembly. In some embodiments, the side port pathway of the catheter assembly may include one or more features to direct fluid flow and induce turbulent fluid flow within the catheter assembly, which may facilitate flushing. In some embodiments, the features of the side port pathway may facilitate flushing of a region within the catheter assembly that may otherwise be difficult to flush free of fluids (such as blood or infusates). In some embodiments, the region may be disposed distal to the septum. Stagnant fluid within the region distal to the septum may lead to accumulation of bacteria within the catheter assembly, which may result in infection or removal of the catheter from the patient.

According to the present invention, the catheter assembly includes an extension tube integrated within the side port. A diameter of the side port pathway increases in a distal direction. The catheter assembly further includes a catheter extending distally from the distal end of the catheter adapter. In some embodiments, the catheter may include a peripheral intravenous catheter, a peripherally-inserted central catheter, or a midline catheter. According to the present invention, the catheter assembly includes a septum, which is disposed within the lumen proximal to the side port pathway.

According to the present invention, the side port pathway includes a first side and a second side opposite the first side. The first side is straight. A proximal end of the second side is straight, and a distal end of the second side is angled proximally with respect to the proximal end of the second side. The first side is distal to the second side. The distal end of the second side contacts the septum.

In some embodiments, the septum may include an elastomeric body surrounded by a canister. In some embodiments, the septum and catheter assembly may not include the canister. In some embodiments, the septum may include a one-piece septum, a two-piece septum, or another suitable septum.

It is to be understood that both the foregoing general description and the following detailed description are examples and explanatory and are not restrictive of the invention, as claimed. It should be understood that the various embodiments are not limited to the arrangements and instrumentality illustrated in the drawings. It should also be understood that the embodiments may be combined, or that other embodiments may be utilized and that structural changes, unless so claimed, may be made without departing from the scope of the various embodiments of the present invention. The following detailed description is, therefore, not to be taken in a limiting sense.

### BRIEF DESCRIPTION OF THE DRAWINGS

Example embodiments will be described and explained with additional specificity and detail through the use of the accompanying drawings in which:
Figure 1 is a cross-sectional view of a prior art catheter assembly;
Figure 2 is a cross-sectional view of an example catheter assembly, illustrating a diameter of an example side port pathway increasing in a distal direction, according to some embodiments;
Figure 3 is a cross-sectional view of the catheter assembly, illustrating an example diverging feature to induce rotational flow, not being part of the present invention;
Figure 4A is a cross-sectional view of the catheter assembly, illustrating example grooves, not being part of the present invention;
Figure 4B is a transverse cross-sectional view of the catheter assembly through the grooves, not being part of the present invention;
Figure 5 is a cross-sectional view of the catheter assembly, illustrating an example helical groove, not being part of the present invention;
Figure 6 is a cross-sectional view of the catheter assembly, illustrating example ridges, not being part of the present invention; and
Figure 7 is a cross-sectional view of the catheter assembly, illustrating an example narrowed diameter portion, not being part of the present invention;
Figure 8A is a cross-sectional view of the catheter assembly, illustrating an example insert, not being part of the present invention;
Figure 8B is a partial cutaway view of the catheter assembly, illustrating the insert of Figure 8A, not being part of the present invention;
Figure 9A is a partial cutaway view of the catheter assembly, illustrating another example insert, not being part of the present invention;
Figure 9B is a cross-sectional view of the catheter assembly through an example side port pathway proximate the insert of Figure 9A, not being part of the present invention;
Figure 10A is a cross-sectional view of the catheter assembly, illustrating another example insert, not being part of the present invention;
Figure 10B is a partial cutaway view of the catheter assembly, illustrating the insert of Figure 10A, not being part of the present invention;
Figure 11A is a cross-sectional view of the catheter assembly, illustrating another example insert, not being part of the present invention;
Figure 11B is a partial cutaway view of the catheter assembly, illustrating the insert of Figure 11A, not being part of the present invention;
Figure 12A is a partial view of the catheter assembly, illustrating another example insert, not being part of the present invention;
Figure 12B is a cross-sectional view of the catheter assembly through an example side port pathway and the insert of Figure 12A, not being part of the present invention;
Figure 13 is a cross-sectional view of the catheter assembly, illustrating another example insert, not being part of the present invention;
Figure 14A is a cross-sectional view of the catheter assembly, illustrating an example rotation element, not being part of the present invention;
Figure 14B is an upper perspective view of the rotation element of Figure 14A, not being part of the present invention;
Figure 14C is a cross-sectional view of the catheter assembly, illustrating another example rotation element, not being part of the present invention;
Figure 14D is a partial cutaway view of the catheter assembly, illustrating the other rotation element, not being part of the present invention;
Figure 15A is a cross-sectional view of the catheter assembly, illustrating an example pivot element, not being part of the present invention; and
Figure 15B is a cross-sectional view of the catheter assembly, illustrating another example pivot element, not being part of the present invention.

### DETAILED DESCRIPTION

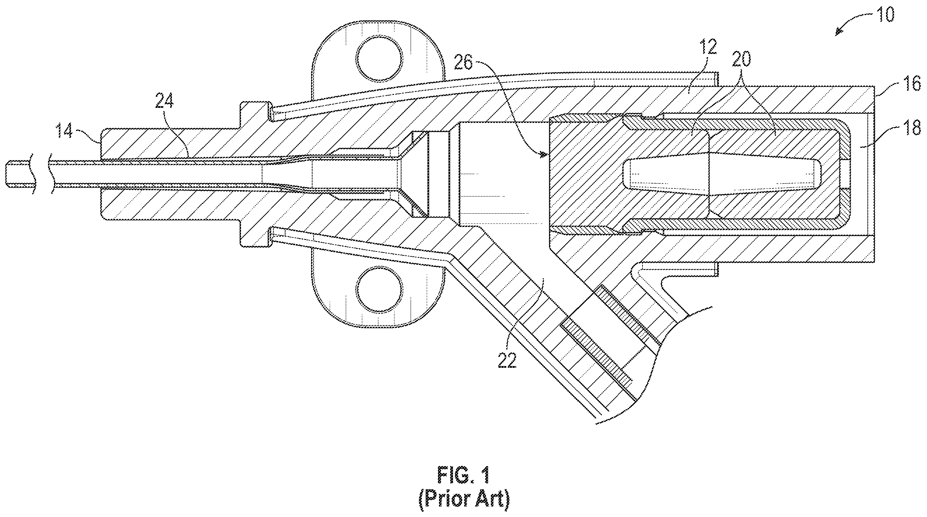

The present disclosure relates generally to vascular access devices, and systems. More particularly, the present disclosure relates to a catheter assembly, as well as related devices. Referring now to Figure 1, a prior art catheter assembly 10 is illustrated. The prior art catheter assembly 10 includes a catheter adapter 12 with a distal end 14, a proximal end 16, and a lumen 18 extending through the distal end 14 and the proximal end 16. The prior art catheter assembly 10 includes a septum 20 spaced apart and proximal to a side port pathway 22. A catheter 24 extends distally from the distal end 14. The prior art catheter assembly includes a region 26 in which fluid may become stagnant despite flushing through the side port pathway 22. The region 26 includes a distal face of the septum 20 and an area within the lumen 18 adjacent thereto. Stagnant fluid within the region 26 may lead to an accumulation of bacteria within the prior art catheter assembly 10, which may result in infection or removal of the catheter 24 from the patient.

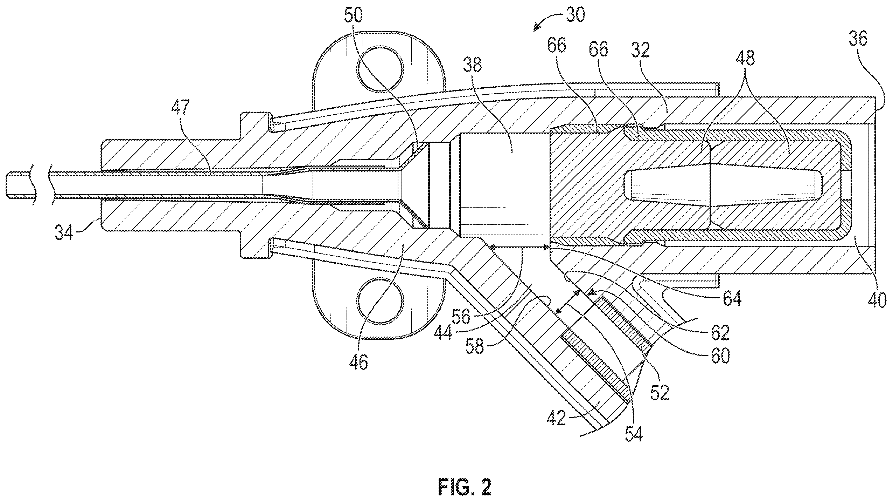

Referring now to Figure 2, a catheter assembly 30 is illustrated, according to some embodiments. According to the present invention, the catheter assembly 30 includes a catheter adapter 32, which includes a distal end 34, a proximal end 36, and an inner surface 38 extending through the distal end 34 and the proximal end 36. The inner surface of the catheter adapter 32 forms a lumen 40. The catheter adapter 32 includes a side port 42 forming a side port pathway 44 through a sidewall 46 of the catheter adapter 32 and in fluid communication with the lumen 40. In some embodiments, the sidewall 46 may include the inner surface 38. In some embodiments, the side port pathway 44 may extend from the lumen 40 to an outer opening of the side port 42.

According to the present invention, the catheter assembly 30 includes a catheter 47 extending distally from the distal end 34 of the catheter adapter 32. In some embodiments, the catheter 47 may include a peripheral intravenous catheter, a peripherally-inserted central catheter, or a midline catheter. In some embodiments, the catheter assembly 30 may include a wedge 50, which may secure the catheter 47 within the distal end 34 of the catheter adapter 32. According to h epresent invention, the catheter assembly 30 includes a septum 48, which is disposed within the lumen 40 proximal to the side port pathway 44.

In some embodiments, the catheter assembly 30 may facilitate flushing of the catheter assembly 30 to avoid stagnant fluid within the catheter assembly 30. In some embodiments, the side port pathway 44 of the catheter assembly 30 may include one or more features to direct fluid flow and induce turbulent fluid flow within the catheter assembly 30, which may facilitate flushing. According to the present invention, the features of the side port pathway 44 facilitate flushing of a region within the catheter assembly 30 that may otherwise be difficult to flush free of fluids (such as blood or infusates). According to the present invention, the region is disposed distal to the septum 48 and may in addition include a region proximate the septum 48. Stagnant fluid within the region distal to the septum 48 may lead to accumulation of bacteria within the catheter assembly 30, which may result in infection or removal of the catheter from the patient. However, the features of the side port pathway 44 may facilitate fluid turbulence within the region, decreasing a likelihood of accumulation of bacteria. In some embodiments, the catheter assembly 30 may reduce build-up of blood on the inner surface 38 and/or reduce interactions of incompatible fluids.

According to the present invention, the catheter assembly includes an extension tube 52 integrated within the side port 42. In some embodiments, the side port pathway 44 may be used for fluid infusion and/or blood collection. In some embodiments, a proximal end of the extension tube 52 may be coupled to a blood collection device.

According to the present invention, a diameter of the side port pathway 44 increases in a distal direction. For example, a proximal portion of the side port pathway 44 may include a first diameter 54, and a distal portion of the side port pathway 44 may include a second diameter 56, which may be greater than the first diameter 54.

According to the present invention, the side port pathway 44 includes a first side 58 and a second side 60 opposite the first side 58. The first side 58 is straight or a have a linear cross-section, as illustrated, for example, in Figure 2. A proximal end 62 of the second side 60 os straight, and a distal end 64 of the second side 60 is angled proximally with respect to the proximal end 62 of the second side 60. Thus, in some embodiments, the second side 60 may be diverging, which may facilitate flushing of the region in front or distal to the septum 48 that may otherwise be difficult to flush free of fluids. According to the present invention, the first side 58 is distal to the second side 60. In some embodiments, the distal end 64 of the second side 60 may be angled between 0° and 90° with respect to the proximal end 62 of the second side 60. In some embodiments, the distal end 64 of the second side 60 may be angled between 20° and 60°, inclusive, with respect to the proximal end 62 of the second side 60. In the invention, the distal end 64 of the second side 60 contacts the septum 48. In some embodiments, a distal end of the first side 58 may be diverging and angled distally with respect to a proximal end of the first side 58, which may increase flushability of the region.

In some embodiments, the septum 48 may include an elastomeric body surrounded by a canister 66. In some embodiments, the septum 48 and catheter assembly 30 may not include the canister 66. In some embodiments, the septum 48 may include a one-piece septum, a two-piece septum, or another suitable septum.

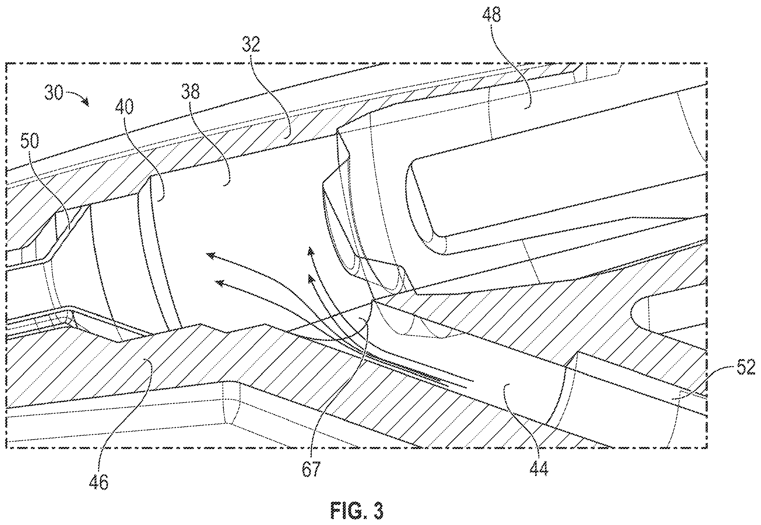

Referring now to Figure 3, in some configurations, a wall of the side port pathway may include a diverging feature, which may induce rotational flow of fluid flowing into the lumen 40 from the side port pathway 44 and facilitate flushing of the region in front or distal to the septum 48. In some configurations, the diverging feature may include a protrusion disposed at an intersection of the side port pathway 44 and the lumen 40. In some configurations, the protrusion may include a stepped surface or wall 67 that may extend across a width of the side port pathway 44.

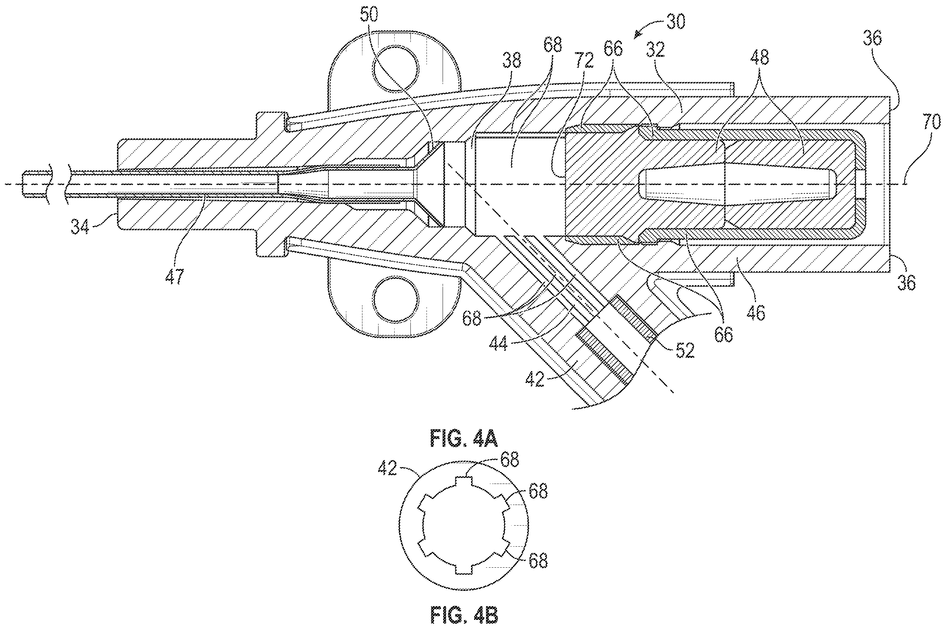

Referring now to Figures 4A-4B, in some configurations, the side port pathway 44 may include a non-cylindrical portion. In some configurations, the non-cylindrical portion may include multiple grooves 68. In some configurations, the grooves 68 may be generally parallel to a central axis of the side port pathway 44, which may intersect a longitudinal axis 70 of the catheter assembly 30. In some configurations, the grooves 68 may be evenly spaced around an inner circumference of the side port 42. In some configurations, each of the grooves 68 may include a distal end proximate or adjacent the lumen 40 and/or a distal end of the septum 48. In some configurations, each of the grooves 68 may include a proximal end proximate or adjacent the extension tube 52. In some configurations, the grooves 68 may be replaced with ribs. In some configurations, the grooves 68 or the ribs may direct fluid flow to facilitate flushing of the region distal to the septum 48. In some configurations, the grooves 68 may be molded using a finned core pin.

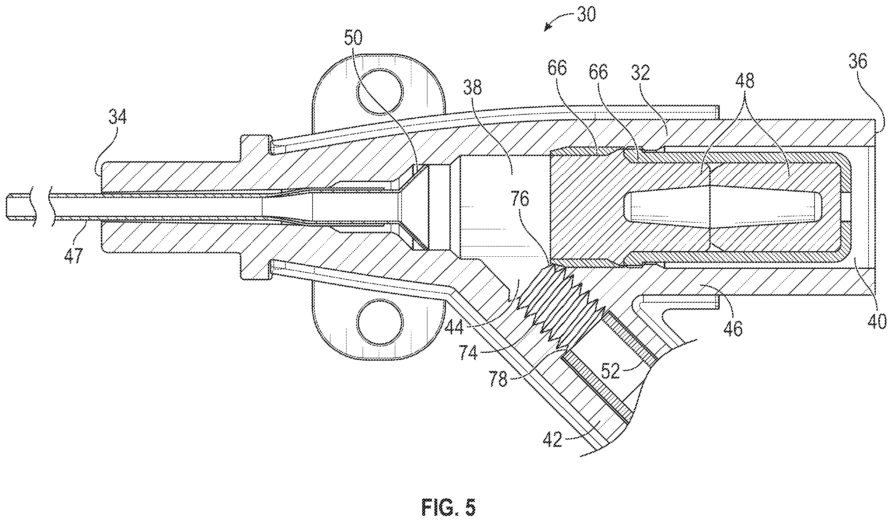

Referring now to Figure 5, in some configurations, the side port pathway 44 may include a non-cylindrical portion. In some configurations, the non-cylindrical portion may include a helical groove 74. In some configurations, the helical groove 74 may wind helically around the inner surface 38, which may be generally cylindrical. In some configurations, a distal end 76 of the helical groove 74 may be proximate the septum 48. In some configurations, the catheter assembly 30 may include the extension tube 52 integrated with the side port 42, and a proximal end 78 of the helical groove 74 may be proximate the extension tube 52.

In some configurations, the helical groove 74 may facilitate flushability by inducing more turbulent fluid flow that increases fluid exchange. In some configurations, the helical groove 74 may terminate just distal of the septum 48, creating a fluid path to the region difficult to flush. In some configurations, the side port 42 may be molded with a threaded core pin. In some configurations, in response to the threaded core pin being screwed or unscrewed during molding, the helical groove 74 may be created.

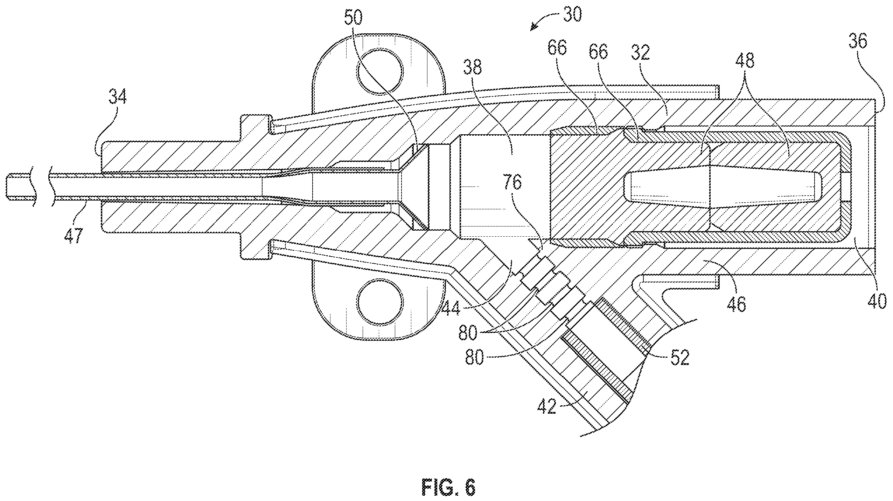

Referring now to Figure 6, in some configurations, the non-cylindrical portion may include multiple ridges 80, which may be generally perpendicular to a central axis of the side port pathway 44. In some configurations, the ridges 80 may be evenly spaced apart. In some configurations, the ridges 80 may be annular. In some configurations, the ridges 80 may increase pressure and restrict flow, resulting in pulsatile or more turbulent fluid flow.

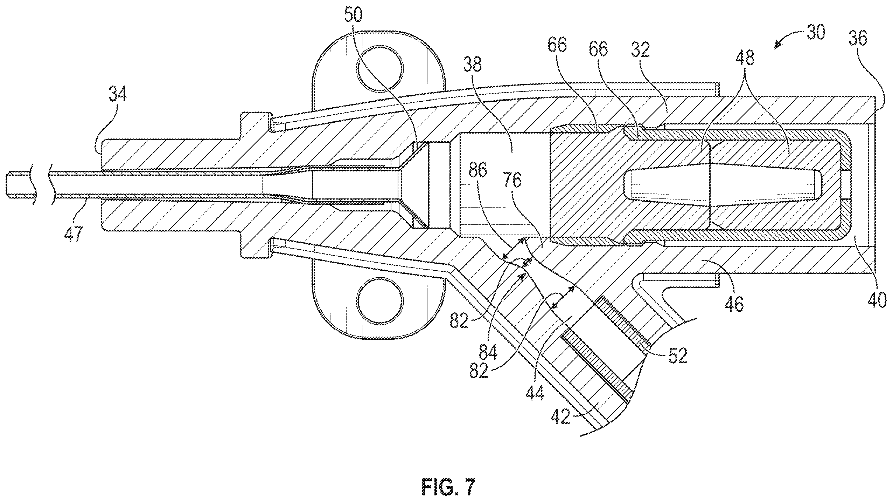

Referring now to Figure 7, in some configurations, the non-cylindrical portion may include a narrowed diameter portion 82. In some configurations, the narrowed diameter portion 82 may function as an inlet nozzle. In some configurations, distal to the narrowed diameter portion 82, the side port pathway 44 may converge in a proximal direction towards the narrowed diameter portion 82, and proximal to the narrowed diameter portion 82, the side port pathway 44 may diverge in the proximal direction away from the narrowed diameter portion 82. In some configurations, the narrowed diameter portion 82 may include a first diameter 84. In some configurations, the side port pathway 44 may include a second diameter 86 distal to the first diameter 84 and a third diameter 88 proximal to the first diameter 84. In some configurations, the second diameter 86 and the third diameter 88 may be greater than the first diameter 84. In some configurations, the narrowed diameter portion 82 may be annular.

In some configurations, the narrowed diameter portion 82 may be proximate the lumen 38. In some configurations, the narrowed diameter portion 82 may be asymmetric. For example, the narrowed diameter portion 82 may include a protrusion only on a distal side of the side port pathway 44. In these and other configurations, the narrowed diameter portion 82 may facilitate turbulence and a rotational flow and deflection toward the septum 48.

In some configurations, the non-cylindrical portion may be disposed within the extension tube 52, which may be disposed within the side port pathway 44 and/or may extend to the lumen 40. Thus, in some configurations, the narrowed diameter portion 82 may be disposed within the extension tube 52. In some configurations, the extension tube 52 may not extend into the lumen 40.

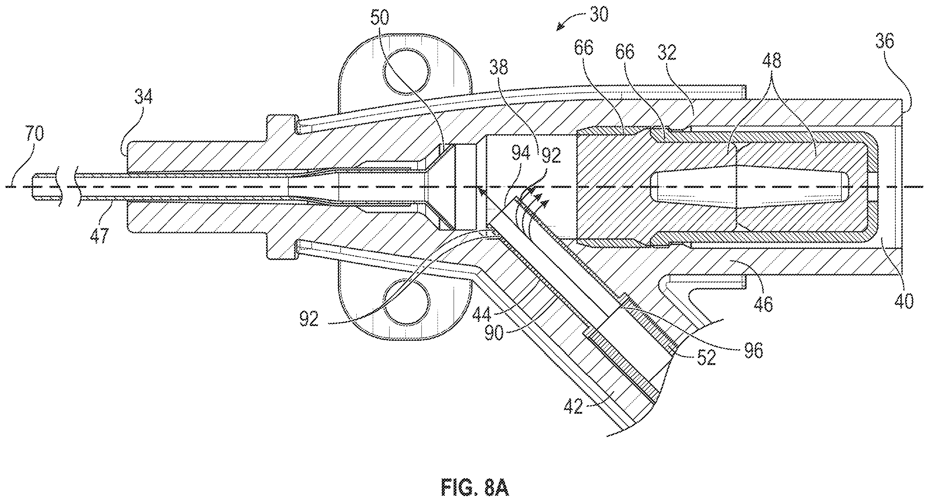

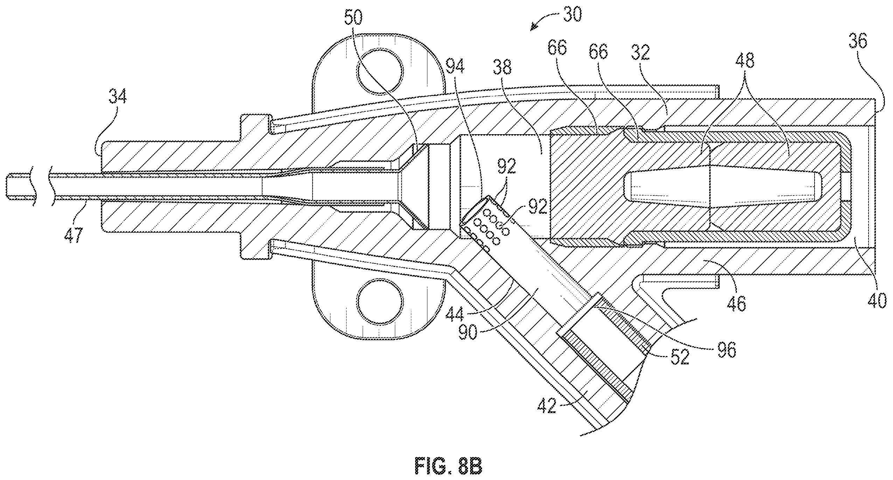

Referring now to Figures 8A-8B, in some configurations, the catheter assembly 30 may include an insert 90 secured within the side port pathway 44 and configured to divert fluid flow. In some configurations, the insert 90 may include a fluid pathway therethrough and in fluid communication with the side port pathway 44 and the lumen 40. In some configurations, the insert 90 may extend into the lumen 40 from the side port pathway 44 and may include one or more perforations or holes 92. In some configurations, the holes 92 may be configured to divert fluid flow towards the septum 48 and into the region difficult to flush.

In some configurations, a distal end of the insert 90 disposed within the lumen 40 may include the holes 92. In some configurations, a proximal end 96 of the insert 90 may be adjacent and/or contacting the extension tube 52. In some configurations, the insert 90 may be tubular or conical. In some configurations, the insert 90 may include an opening 94 aligned with the central axis of the side port pathway 44 and/or larger than the holes 92 such that a significant portion of the fluid continues in the distal direction. In some configurations, the term "insert" may refer to an element that is inserted within the catheter adapter 32 and is not monolithically formed with the catheter adapter as a single unit. In some configurations, the insert 90 may be formed by a distal end of the extension tube 52, which may extend into or through the side port pathway 44 and which may divert fluid flow.

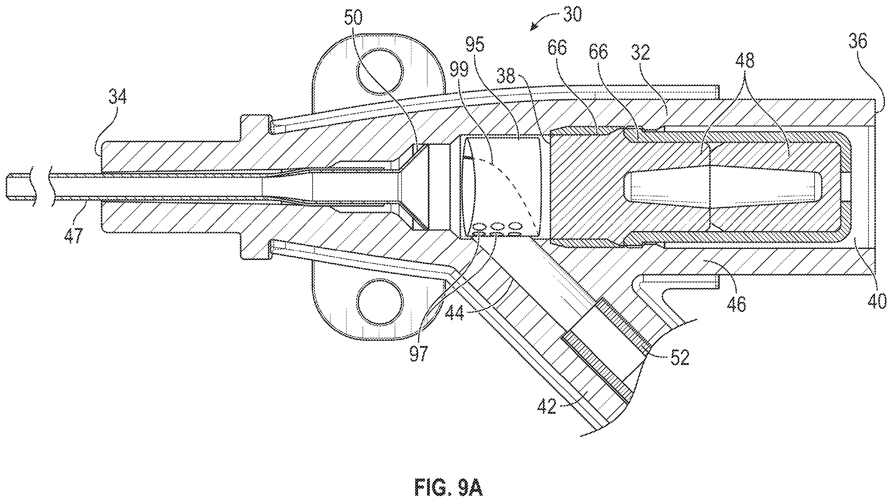

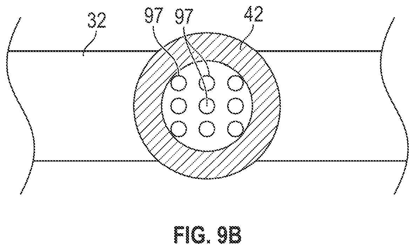

Referring now to Figures 9A-9B, in some configurations, an insert 95 may include a ring, which may include one or more holes 97 aligned with the side port pathway 44. In some configurations, the holes 97 may be configured to divert fluid flow, which may flow towards the septum 48. In some configurations, the ring may include a channel therethrough such that an introducer needle (not illustrated) may extend through the ring and the catheter 47. In some configurations, the channel may be aligned with the longitudinal axis 70 (see, for example, Figure 4A) of the catheter assembly 30. In some configurations, an inner surface of the ring may include a groove or a ridge 99, which may guide an instrument inserted through the side port 42, a particular one of the holes 97, and into and/or through the catheter 47. In some configurations, the instrument may include a probe, obturator, introducer, catheter, or another suitable instrument.

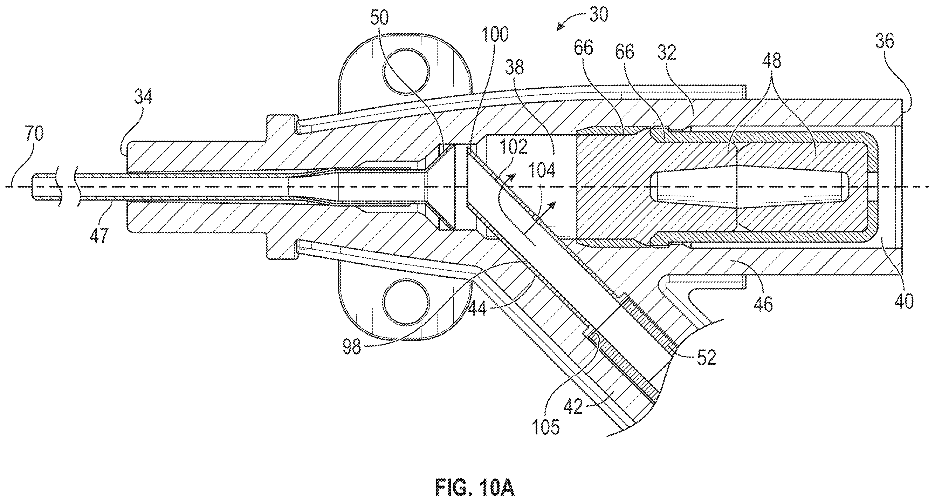

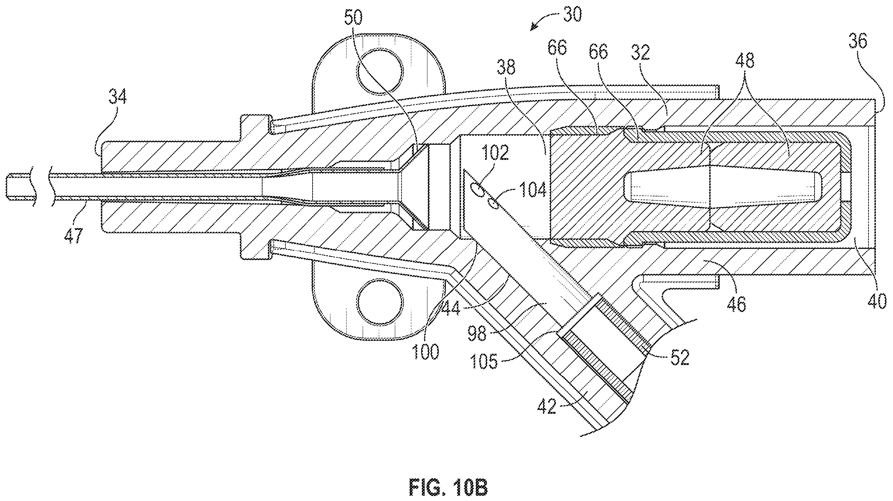

Referring now to Figures 10A-10B, in some configurations, the catheter assembly 30 may include an insert 98 secured within the side port pathway 44 and configured to divert fluid flow. In some configurations, the insert 98 may include a fluid pathway therethrough and in fluid communication with the side port pathway 44 and the lumen 40. In some configurations, the insert 98 may extend into the lumen 40 from the side port pathway 44. In some configurations, the insert 98 may include a cannula, which may include a distal end generally perpendicular to the longitudinal axis 70 (see, for example, Figure 4A) of the catheter assembly 30. In some configurations, the distal end of the insert 98 may include an opening 100 and may be configured to direct fluid distally.

In some configurations, the cannula may include a first side hole 102 and a second side hole 104. In some configurations, the first side hole 102 may be aligned with the opening 100, and the first side hole 102 and the opening 100 may be configured to receive the introducer needle therethrough. In some configurations, the second side hole 104 may be configured to direct fluid from the side port 42 proximally toward the septum 48 and the region difficult to flush. In some configurations, the second side hole 104 may be proximal to the first side hole 102. In some configurations, a proximal end 105 of the insert 98 may be adjacent and/or contacting the extension tube 52.

In some configurations, the insert 98 may be formed by a distal end of the extension tube 52, which may extend into or through the side port pathway 44 and which may divert fluid flow. Thus, in some configurations, the insert 98 and the extension tube 52 may be monolithically formed as a single unit.

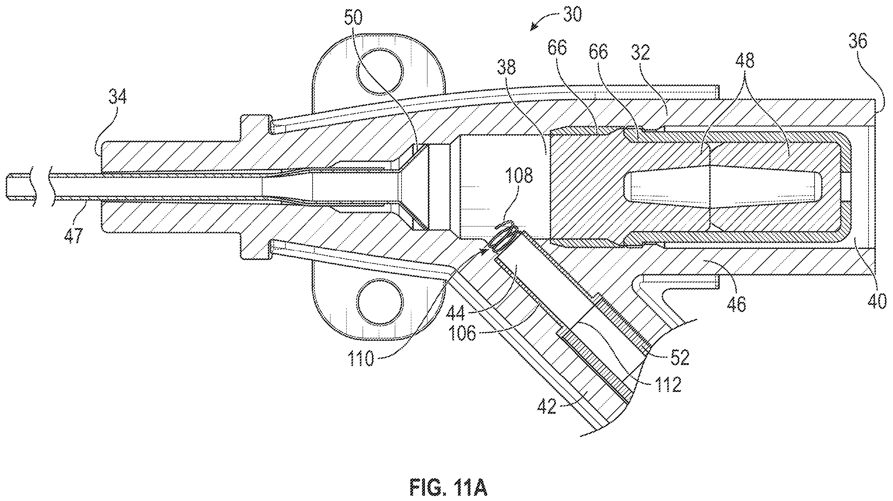

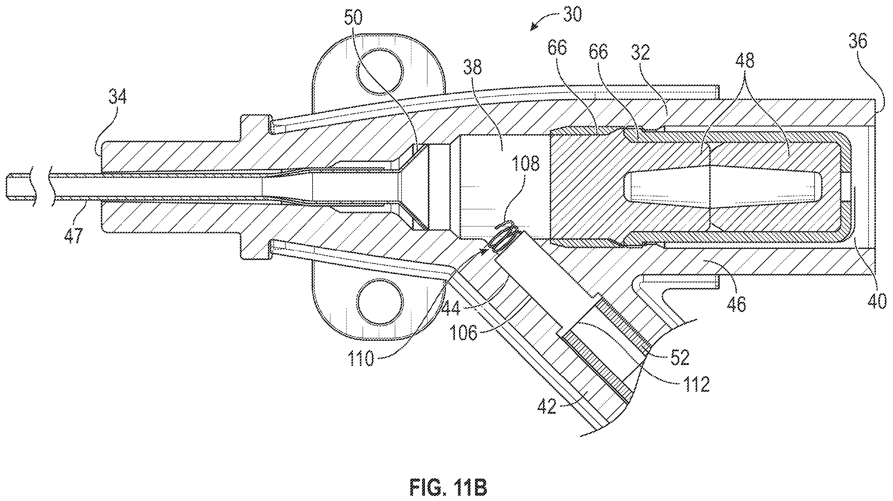

Referring now to Figures 11A-11B, in some configurations, the catheter assembly 30 may include an insert 106 secured within the side port pathway 44. In some configurations, a distal end 110 of the insert 106 may include a corkscrew shape 108, which may be helical. In some configurations, the corkscrew shape 108 may include multiple coils, which may be disposed within the lumen 40 and provide a guide for the instrument inserted through the side port 42. In some configurations, the corkscrew shape 108 may divert fluid flow from the side port pathway 44 and may facilitate flushing of the region. In some configurations, the insert 106 may include a fluid pathway therethrough and in fluid communication with the side port pathway 44 and the lumen 40. In some configurations, the insert 106 may extend into the lumen 40 from the side port pathway 44. In some configurations, the insert 106 may include a cannula, and the corkscrew shape 108 may extend from a distal end of the cannula. In some configurations, the distal end of the cannula may include an opening and may be configured to direct fluid distally. In some configurations, a proximal end 112 of the insert 106 may be adjacent and/or contacting the extension tube 52.

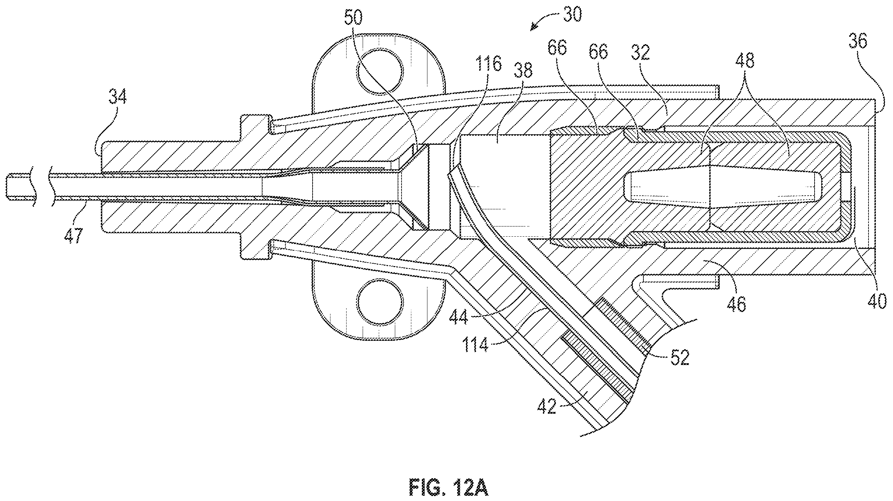

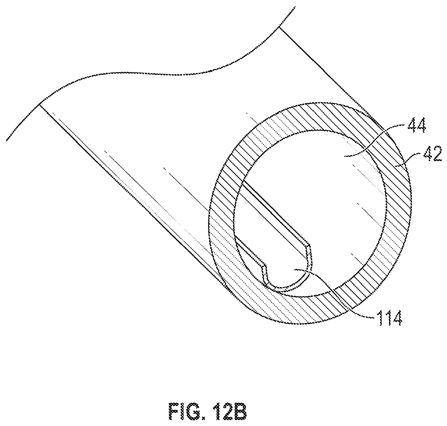

Referring now to Figures 12A-12B, in some configurations, the catheter assembly 30 may include an insert 114, which may include a groove extending along a longitudinal axis of the insert 114. In some configurations, the groove may be semi-circular.

In some configurations, the insert 114 may be advanced through the side port 42 and into the catheter 47 immediately prior to flushing. In some configurations, during flushing, the groove may direct fluid flow toward the septum 48 and the region otherwise difficult to flush. In some configurations, after completion of flushing, the insert 114 may be removed from the catheter assembly 30 so as to not interfere with infusion or aspiration.

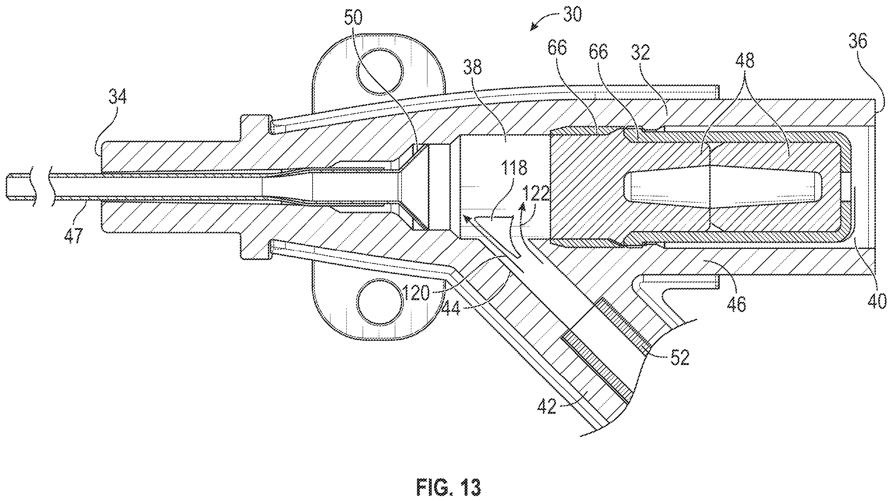

Referring now to Figure 13, in some configurations, the catheter assembly may include a insert 118 that may be coupled to the inner surface 38. In some configurations, the insert 118 may include a protrusion, which may include a generally triangular shape. In some configurations, the protrusion may be monolithically formed with the inner surface 38 as a single unit instead of being inserted into the lumen 40. In some configurations, the protrusion may divide the side port pathway 44 into a first fluid pathway 120 extending in a distal direction and a second fluid pathway 122 extending in a proximal direction towards the septum 48.

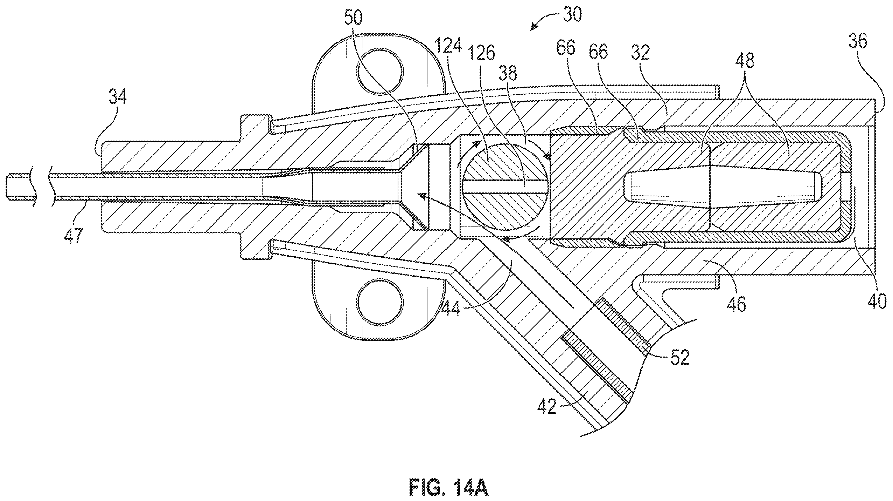

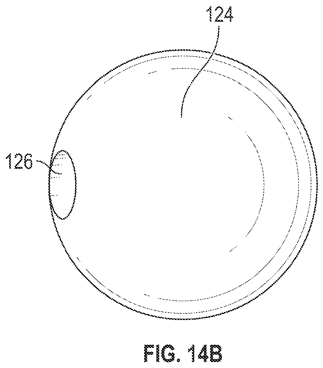

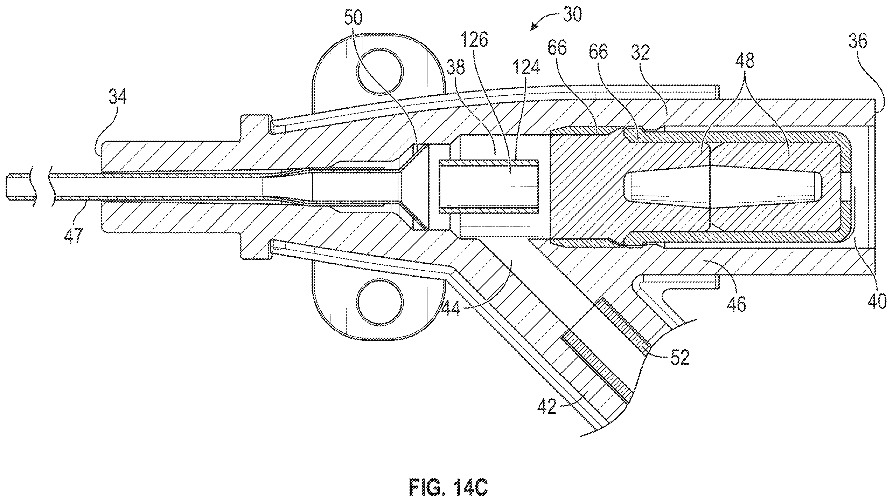

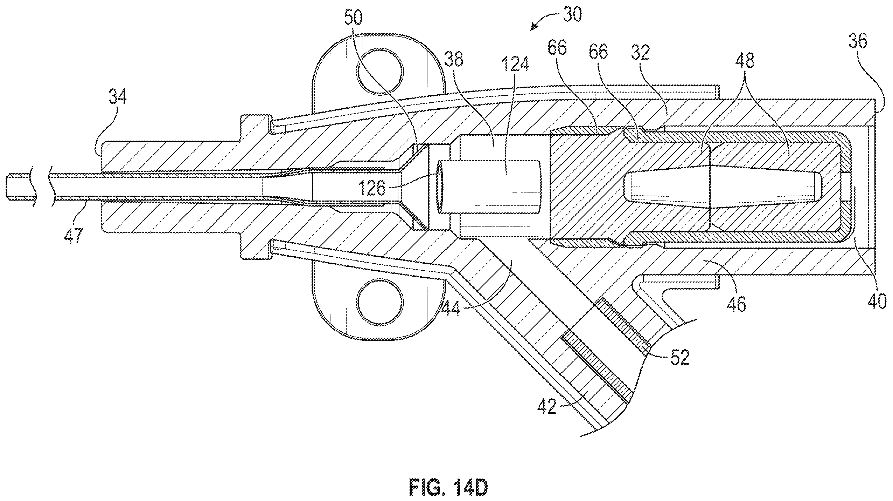

Referring now to Figures 14A-14D, in some configurations, the catheter assembly 30 may include a fluid diverter 124 disposed between the septum 48 and the catheter 47 and aligned with the side port pathway 44. In some configurations, the fluid diverter 124 may include a rotation element, which may include a straight pathway 126 extending therethrough. In some configurations, the introducer needle may extend through the straight pathway 126, which may limit rotation of the rotation element. In some configurations, the rotation element may be a cylinder, as illustrated, for example, in Figures 14C-14D, or a sphere, as illustrated, for example in Figures 14A-14B. In some configurations, in response to the introducer needle being removed from the catheter assembly 30, the rotation element may be configured to freely rotate or spin in response to infusion of fluid from the side port pathway 44 to the lumen 40, redirecting fluid and improving flushability. In some configurations, the rotation element may be configured to float in the fluid in the lumen 40. In some configurations, the rotation element may not be coupled to the catheter adapter 32 and may be unfixed. In some configurations, the rotation element may be coated with an anti-microbial coating to prevent bacterial growth within the catheter adapter 32.

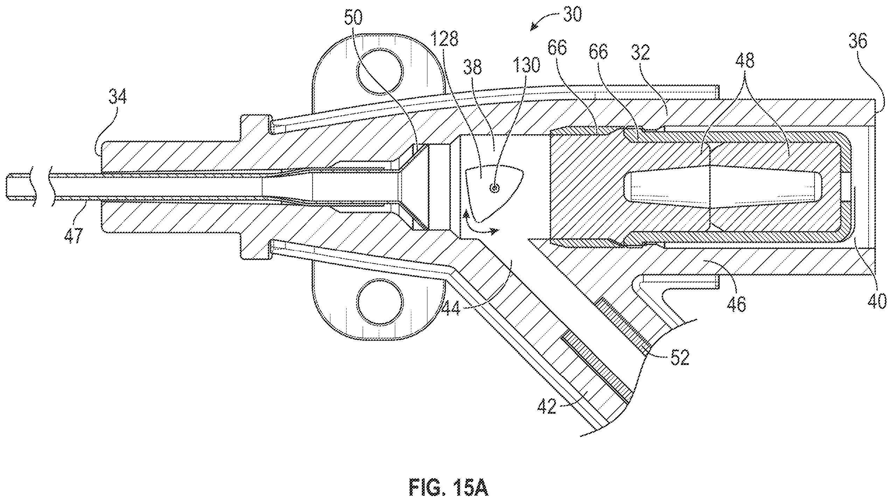

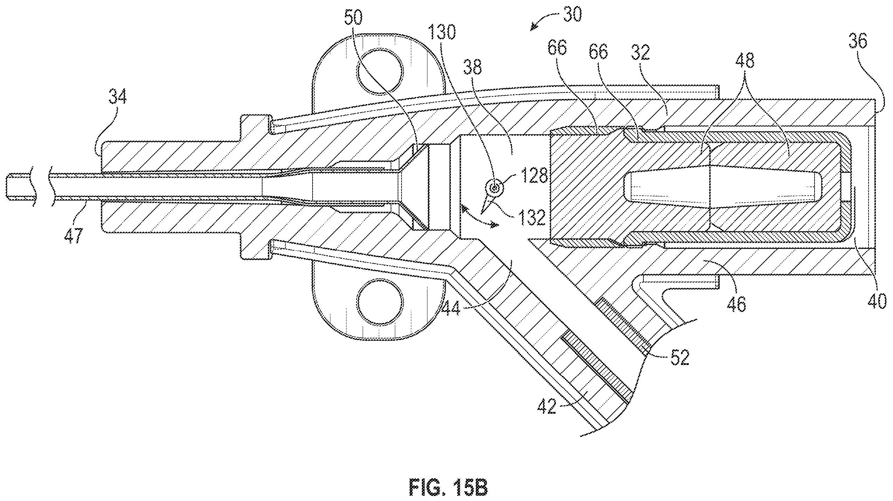

Referring now to Figures 15A-16B, in some configurations, a fluid diverter may include a pivot element 128 coupled to the inner surface 38 and configured to pivot. In some configurations, the pivot element 128 may pivot about a pin 130, which may couple the pivot element 128 to the inner surface 38. In some configurations, the pivot element 128 may be generally triangular shaped, as illustrated, for example, in Figures 15A-15B. As illustrated in Figures 16A-16B, the pivot element 128 may include an elongated tab 132 extending outwardly from a body of the pivot element 128. In some configurations, the pivot element 128 may include a door or opening to allow a separate device, such as the instrument, to be passed through. In some configurations, the pivot element 128 may be configured to pivot in response to insertion of the instrument or other device distally through the side port 42.

All examples and conditional language recited herein are intended for pedagogical objects to aid the reader in understanding the invention and the concepts contributed by the inventor to furthering the art and are to be construed as being without limitation to such specifically recited examples and conditions. Although embodiments of the present inventions have been described in detail, it should be understood that the various changes, substitutions, and alterations could be made hereto without departing from the scope of the invention.

## Claims

1. A catheter assembly (30), comprising:
a catheter adapter (32), comprising a distal end (34), a proximal end (36), an inner surface (38) extending through the distal end (34) and the proximal end (36) and forming a lumen (40), and a side port (42) forming a side port pathway (44) through a sidewall (46) of the catheter adapter (32) and in fluid communication with the lumen (40);
an extension tube (52) having a distal end positioned in the side port (42), wherein a diameter of the side port pathway (44) increases in a distal direction;
a catheter (47) extending distally from the distal end (34) of the catheter adapter (32); and
a septum (48) disposed within the lumen (40) proximal to the side port pathway (44);
wherein the side port (42) provides for introduction of a fluid flow into the lumen (40) of the catheter adapter (32), with the diameter of the side port pathway (44) increasing in the distal direction to modify the fluid flow into the lumen (40) and thereby facilitate flushing of the lumen (40) in a region thereof distal to the septum (48); and
wherein the side port pathway (44) comprises a first side (58) and a second side (60) opposite the first side (58), wherein the first side (58) is straight, wherein a proximal end (62) of the second side (60) is straight and a distal end (64) of the second side (60) is angled proximally with respect to the proximal end (62) of the second side (60), wherein the first side (58) is distal to the second side (60),
**characterised in that** the distal end (64) of the second side (60) contacts the septum (48).

2. The catheter assembly (30) of claim 1, wherein the septum (48) comprises an elastomeric body surrounded by a canister (66).

## Patentansprüche

1. Katheterbaugruppe (30), die aufweist:
einen Katheteradapter (32), der ein distales Ende (34), ein proximales Ende (36), eine Innenfläche (38), die sich durch das distale Ende (34) und das proximale Ende (36) erstreckt und ein Lumen (40) bildet, und einen Seitenport (42) aufweist, der einen Seitenportweg (44) durch eine Seitenwand (46) des Katheteradapters (32) bildet und in Fluidverbindung mit dem Lumen (40) steht;
einen Verlängerungsschlauch (52) mit einem distalen Ende, das in dem Seitenport (42) positioniert ist, wobei ein Durchmesser des Seitenportwegs (44) in einer distalen Richtung zunimmt;
einen Katheter (47), der sich distal von dem distalen Ende (34) des Katheteradapters (32) erstreckt; und
ein Septum (48), das in dem Lumen (40) proximal zu dem Seitenportweg (44) angeordnet ist;
wobei der Seitenport (42) für die Einführung eines Fluidstroms in das Lumen (40) des Katheteradapters (32) sorgt, wobei der Durchmesser des Seitenportwegs (44) in distaler Richtung zunimmt, um den Fluidstrom in das Lumen (40) zu modifizieren und dadurch das Spülen des Lumens (40) in einem Bereich davon distal zum Septum (48) zu erleichtern; und
wobei der Seitenportweg (44) eine erste Seite (58) und eine zweite Seite (60) entgegengesetzt zur ersten Seite (58) aufweist, wobei die erste Seite (58) gerade ist, wobei ein proximales Ende (62) der zweiten Seite (60) gerade ist und ein distales Ende (64) der zweiten Seite (60) proximal in Bezug auf das proximale Ende (62) der zweiten Seite (60) abgewinkelt ist, wobei die erste Seite (58) distal zu der zweiten Seite (60) ist, **dadurch gekennzeichnet, dass** das distale Ende (64) der zweiten Seite (60) das Septum (48) berührt.

2. Katheterbaugruppe (30) nach Anspruch 1, wobei das Septum (48) einen Elastomerkörper aufweist, der von einem Kanister (66) umgeben ist.

## Revendications

1. Ensemble cathéter (30), comprenant :
un adaptateur de cathéter (32), comprenant une extrémité distale (34), une extrémité proximale (36), une surface interne (38) s'étendant à travers l'extrémité distale (34) et l'extrémité proximale (36) et formant une lumière (40), et un orifice latéral (42) formant un trajet d'orifice latéral (44) à travers une paroi latérale (46) de l'adaptateur de cathéter (32) et en communication fluidique avec la lumière (40) ;
un tube d'extension (52) présentant une extrémité distale positionnée dans l'orifice latéral (42), dans lequel un diamètre du trajet d'orifice latéral (44) augmente dans une direction distale ;
un cathéter (47) s'étendant de façon distale à partir de l'extrémité distale (34) de l'adaptateur de cathéter (32) ; et
un septum (48) disposé à l'intérieur de la lumière (40) proximal au trajet d'orifice latéral (44) ;
dans lequel l'orifice latéral (42) est prévu pour l'introduction d'un écoulement de fluide dans la lumière (40) de l'adaptateur de cathéter (32), le diamètre du trajet d'orifice latéral (44) augmentant dans la direction distale pour modifier l'écoulement de fluide dans la lumière (40) et faciliter ainsi le rinçage de la lumière (40) dans une région de celle-ci distale par rapport au septum (48) ; et
dans lequel le trajet d'orifice latéral (44) comprend un premier côté (58) et un second côté (60) opposé au premier côté (58), dans lequel le premier côté (58) est droit, dans lequel une extrémité proximale (62) du second côté (60) est droite et une extrémité distale (64) du second côté (60) est inclinée de façon proximale par rapport à l'extrémité proximale (62) du second côté (60), dans lequel le premier côté (58) est distal par rapport au second côté (60),
**caractérisé en ce que** l'extrémité distale (64) du second côté (60) est en contact avec le septum (48).

2. Ensemble cathéter (30) selon la revendication 1, dans lequel le septum (48) comprend un corps élastomère entouré d'un réservoir (66).
